# EUROPEAN PATENT SPECIFICATION

(11) **EP 3 148 611 B1**
(45) Date of publication and mention of the grant of the patent: **02.12.2020**
(21) Application number: 15799090.4
(22) Date of filing: 29.05.2015
(51) Int. Cl.: G16H 20/17, G16H 40/63, A61M 5/168, A61M 5/172

(54) **INFUSION SYSTEM AND PUMP WITH CONFIGURABLE CLOSED LOOP DELIVERY RATE CATCH-UP**
INFUSIONSSYSTEM UND PUMPE MIT KONFIGURIERBARER AUSGABERATENAUFHOLUNG DES GESCHLOSSENEN REGELKREISES
SYSTÈME ET POMPE DE PERFUSION À RATTRAPAGE DE DÉBIT D'ADMINISTRATION RÉGLABLE EN BOUCLE FERMÉE

(30) Priority: 29.05.2014 US 201462004688 P
(43) Date of publication of application: 05.04.2017
(73) Proprietor: ICU Medical, Inc., San Clemente, CA 92673 (US)
(72) Inventor: LINDO, Steve, Chicago, Illinois 60641 (US); DAY, William, Hoffman Estates, Illinois 60192 (US)
(74) Representative: Invent Horizon IP
(86) International application number: PCT/US2015/033345
(87) International publication number: WO 2015/184366

(56) References cited:
- US-A- 4 525 163
- US-A- 5 522 798
- US-A1- 2002 077 852
- US-A1- 2002 077 852
- US-A1- 2006 064 053
- US-A1- 2006 270 971
- US-A1- 2006 270 971
- US-A1- 2009 153 058

## Description

### TECHNICAL FIELD

The present invention relates to medical devices. More specifically, the invention relates to infusion systems and pumps with configurable closed loop delivery rate catch-up.

### BACKGROUND OF THE INVENTION

Infusion pumps are medical devices that deliver fluids, including nutrients and medications such as antibiotics, chemotherapy drugs, and pain relievers, into a patient's body in controlled amounts. Many types of pumps, including large volume, patient-controlled analgesia (PCA), elastomeric, syringe, enteral, and insulin pumps, are used worldwide in healthcare facilities such as hospitals, and in the home. Clinicians and patients rely on pumps for safe and accurate administration of fluids and medications.

Presently available infusion pumps use an open loop pumping rate: the desired pumping or volumetric flow rate is input directly, or calculated from an input volume to be infused and delivery period or duration, and the infusion pump operates at a single target motor speed or stroke frequency to deliver the desired pumping or flow rate regardless of external conditions. Unfortunately, flow delivery can be interrupted by a variety of conditions, such as a stoppage or pause based upon a full or partial occlusion, a kinked tube, an air-in-line alarm, hanging a new IV bag, vein clot, or the like. Once the flow delivery is interrupted, the time in which there is no medication delivery is lost, resulting in a delay in desired infusion completion.

Nurses typically work in shifts and expect certain medications to be started and/or finished within their shift and plan accordingly. When occlusions, pauses, or other disturbances interrupt or delay medication delivery, this disrupts the nurses' planning for patient care within their respective shifts. In addition, the patients in these scenarios would not receive the medicine required within the allotted time.

US 5,522,798 discloses a host controller to controls drug concentrations for each of a plurality of channels delivered through multiple drug channels of a multi-channel drug delivery system. The host controller includes a controller that is coupled to each drug channel of the multichannel drug delivery system to receive actual drug delivery rate information.

US 4,525,163 discloses a controller for an intravenous set which operates both to maintain the instantaneous drip rate through a drip chamber included in the IV set at a desired value, and to modify this value repeatedly during the course of an infusion. The desired drip value is modified in accordance with the measured volumetric flow rate through the intravenous set such that if a particular infusion fluid produces abnormally small volume drops in the drip chamber, then the controller automatically increases the drip rate to compensate.

US 2006/0270971 discloses a patient hydration system including an infusion device for administering hydration fluid to a patient and a patient urine output measurement device. If a disruption is detected, a controller software calculates an amount of urine made by a patient during the disruption using weight measurements, and the system increases the infusion rate.

US 2002/0077852 discloses a system for creating a customized drug library for an electronically loadable drug infusion pump, the system including a drug library containing a plurality of drug entries, there being associated with each drug entry a set of associated drug delivery parameters and/or drug delivery protocols for configuring the drug infusion pump.

US 2006/0064053 discloses an infusion system comprising a first pump device that delivers a first infusate to an IV infusion line and a second pump device that delivers a second infusate to the IV line. A measurement device determines an amount of undelivered first infusate remaining in a receptacle. A control system communicates with the first and second delivery devices and the measurement device to determine an optimum first infusate infusion flow rate based on the determined amount of undelivered first infusate remaining in the system, a predetermined volume of fluid to be infused, and a predetermined infusion time period.

It would be desirable to have an infusion system and pump with configurable closed loop delivery rate catch-up that would overcome the above disadvantages.

### SUMMARY OF THE INVENTION

The scope of the invention is defined by the appended claims. Embodiments and examples not falling within the scope of the claims are for reference only.

Other features and advantages of the invention will become further apparent from the following detailed description of the presently preferred embodiments, read in conjunction with the accompanying drawings. The detailed description and drawings are merely illustrative of the invention rather than limiting, the scope of the invention being defined by the appended claims.

### BRIEF DESCRIPTION OF THE DRAWINGS

**FIG. 1** is a schematic diagram of the medication management system including a medication management unit and a medical device integrated with other systems in a hospital environment, in accordance with the present invention;
**FIG. 2** is a schematic diagram of the medication management unit, in accordance with the present invention;
**FIG. 3** is a schematic diagram of some of the major functions performed by the medication management unit, in accordance with the present invention;
**FIG. 4** is a schematic diagram of a medical device, in accordance with the present invention;
**FIGS. 5A** **&** **5B** are perspective views of a multi-channel medical device, in accordance with the present invention;
**FIGS. 6A** **&** **6B** are screen shots of a graphical user interface and detail of the graphical user interface, respectively, for configuring a drug library, in accordance with the present invention;
**FIG. 7** is a graph of an infusion volume and infusion rate versus time modeled for an infusion with an infusion pump employing configurable closed loop delivery rate catch-up, in accordance with the present invention;
**FIG. 8** is a block diagram of a control model for an infusion pump employing configurable closed loop delivery rate catch-up, in accordance with the present invention;
**FIG. 9** is a flowchart of a method for configuring a drug library for use with an infusion system employing configurable closed loop delivery rate catch-up, in accordance with the present invention; and
**FIG. 10** is a flowchart of a method for operating an infusion pump employing configurable closed loop delivery rate catch-up, in accordance with the present invention.

### DETAILED DESCRIPTION OF PRESENTLY PREFERRED EMBODIMENTS

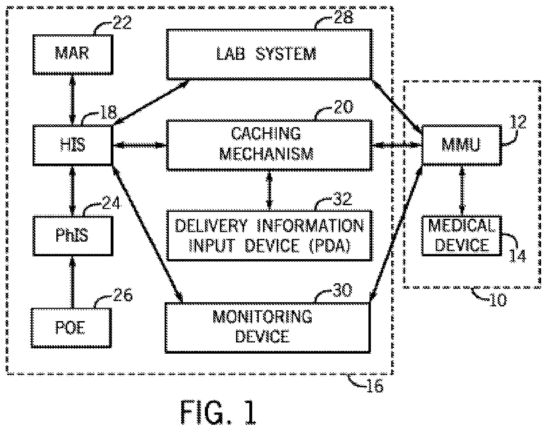

**FIG. 1** is a schematic diagram of the medication management system including a medication management unit and a medical device integrated with an information system, in accordance with the present invention. The medication management system (MMS) **10** includes a medication management unit (MMU) **12** and a medical device **14,** typically operating in a hospital environment **16.** The term hospital environment as defined herein is used broadly to mean any medical care facility, including but not limited to a hospital, treatment center, clinic, doctor's office, day surgery center, hospice, nursing home, and any of the above associated with a home care environment. There can be a variety of information systems in a hospital environment. As shown in **FIG. 1****,** the MMU **12** communicates to a hospital information system (HIS) **18** via a caching mechanism **20** that is part of the hospital environment **16.**

Those skilled in the art will appreciate that the caching mechanism **20** is primarily a pass through device for facilitating communication with the HIS **18** and its functions can be eliminated or incorporated into the MMU **12** (**FIG. 1****)** and/or the medical device **14** and/or the HIS **18** and/or other information systems or components within the hospital environment **16.** The caching mechanism **20** provides temporary storage of hospital information data separate from the HIS **18,** the medication administration record system (MAR) **22,** pharmacy information system (PhIS) **24,** physician order entry (POE) **26,** and/or Lab System **28.** The caching mechanism **20** provides information storage accessible to the medication management system **10** to support scenarios where direct access to data within the hospital environment **16** is not available or not desired. In one example, the caching mechanism **20** provides continued flow of information in and out of the MMU **12** in instances where the HIS **18** is down or the connectivity between the MMU **12** and the electronic network (not shown) is down.

The HIS **18** communicates with a medication administration record system (MAR) **22** for maintaining medication records and a pharmacy information system (PhIS) **24** for delivering drug orders to the HIS. A physician/provider order entry (POE) device **26** permits a healthcare provider to deliver a medication order prescribed for a patient to the hospital information system directly or indirectly via the PhIS **24.** One skilled in the art will also appreciate that a medication order can be sent to the MMU **12** directly from the PhIS **24** or POE device **26.** As used herein, the term medication order is defined as an order to administer something that has a physiological impact on a person or animal, including but not limited to liquid or gaseous fluids, drugs or medicines, liquid nutritional products and combinations thereof.

Lab system **28** and monitoring device **30** also communicate with the MMU **12** to deliver updated patient-specific information to the MMU **12.** As shown, the MMU **12** communicates directly to the lab system **28** and monitoring device **30.** However, those skilled in the art will appreciate that the MMU **12** can communicate with the lab system **28** and monitoring device **30** indirectly via the HIS **18,** the caching mechanism **20,** the medical device **14** or some other intermediary device or system.

Delivery information input device **32** also communicates with the MMU **12** to assist in processing drug orders for delivery through the MMU **12.** The delivery information input device **32** can be any sort of data input means, including those adapted to read machine readable indicia such as barcode labels; for example a personal digital assistant (PDA) with a barcode scanner. Hereinafter, the delivery information input device **32** is referred to as input device **32.** Alternatively, the machine readable indicia can be in other known forms, such as radio frequency identification (RFID) tag, two-dimensional bar code, ID matrix, transmitted radio ID code, human biometric data such as fingerprints, etc. and the input device **32** adapted to "read" or recognize such indicia. The input device **32** is shown as a separate device from the medical device **14;** alternatively, the input device **32** communicates directly with the medical device **14** or can be integrated wholly or in part with the medical device.

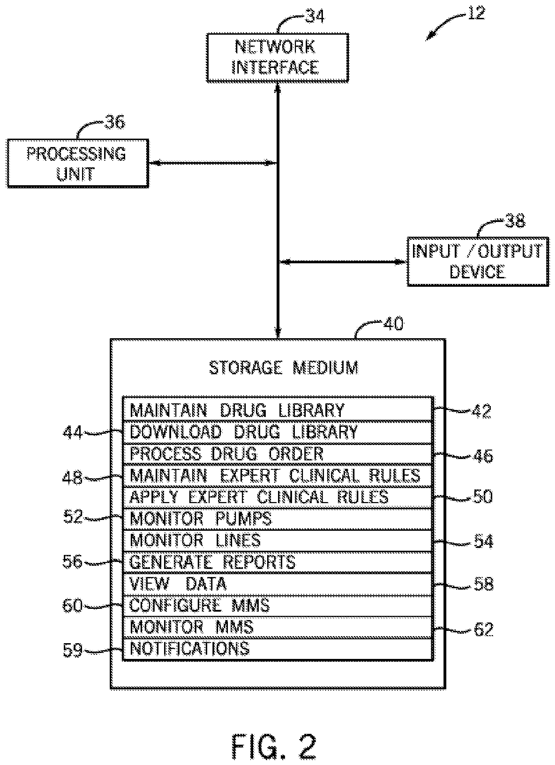

**FIG. 2** is a schematic diagram of the medication management unit, in accordance with the present invention. The medication management unit **12** includes a network interface **34** for connecting the MMU **12** to multiple components of a hospital environment **16,** one or more medical devices **14,** and any other desired device or network. A processing unit **36** is included in MMU **12** and performs various operations described in greater detail below. A display/input or user interface device **38** communicates with the processing unit **36** and allows the user to receive output from processing unit **36** and/or input information into the processing unit **36.** Those skilled in the art will appreciate that the display/input device **38** can be provided as a separate display device and a separate input device.

An electronic storage medium **40** communicates with the processing unit **36** and stores programming code and data necessary for the processing unit **36** to perform the functions of the MMU **12.** More specifically, the storage medium **40** stores multiple programs formed in accordance with the present invention for various functions of the MMU **12** including but not limited to the following programs: Maintain Drug Library **42;** Download Drug Library **44;** Process Drug Order **46;** Maintain Expert Clinical Rules **48;** Apply Expert Clinical Rules **50;** Monitor Pumps **52;** Monitor Lines **54;** Generate Reports **56;** View Data **58;** Configure the MMS **60;** and Monitor the MMS **62.** The Maintain Drug Library **42** program creates, updates, and deletes drug entries and establishes a current active drug library. The Download Drug Library **44** program updates medical devices **14** with the current drug library. The Process Drug Order **46** program processes the medication order for a patient, verifying that the point of care (POC) medication and delivery parameters match those ordered. The Maintain Expert Clinical Rules **48** program creates, updates, and deletes the rules that describe the hospital's therapy and protocol regimens. The Apply Expert Clinical Rules **50** program performs logic processing to ensure safety and considers other infusions or medication orders, patient demographics, and current patient conditions. The Monitor Pumps **52** program acquires ongoing updates of status events, and alarms transmitted both near real-time and in batch mode, as well as tracking the location, current assignment, and software versions such as the drug library version residing on medical device **14.** The Monitor Lines **54** program acquires ongoing updates of status, events and alarms for each channel or line for a medical device **14** that supports multiple drug delivery channels or lines. The Generate Reports **56** program provides a mechanism that allows the user to generate various reports of the data held in the MMU storage medium **40.** The View Data **58** program provides a mechanism that supports various display or view capabilities for users of the MMU **12.** The Notifications **59** program provides a mechanism for scheduling and delivery of events to external systems and users. The Configure the MMS **60** program provides a mechanism for system administrators to install and configure the MMS **10.** The Monitor the MMS **62** program enables information technology operations staff capabilities to see the current status of MMS **10** components and processing, and other aspects of day-to-day operations such as system start up, shut down, backup and restore.

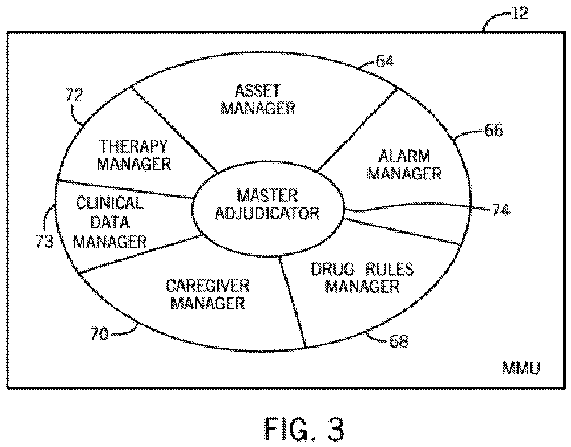

**FIG. 3** is a schematic diagram of some of the major functions performed by the medication management unit, in accordance with the present invention. The various functional programs **42-62** of the MMU **12,** each including separate features and rules, are partitioned (at a higher level than shown in **FIG. 2****)** and logically organized into interrelated managing units of the MMU **12.** As shown, the MMU **12** includes an asset manager **64,** an alarm manager **66,** a drug library manager (such as, for example, is included in HOSPIRA MEDNET™ software) **68,** a caregiver manager **70,** a therapy manager **72,** and/or a clinical data manager **73.** However, those skilled in the art will appreciate that additional or alternative hospital system managing units can be provided without departing from the present invention. Additionally, the MMU **12** includes a master adjudicator **74** between the separate interrelated managing units **64-73** of the MMU **12,** to regulate the interaction between the separate management units.

Further, while the MMU **12** as described herein appears as a single device, there can be more than one MMU **12** operating harmoniously and sharing the same database. For example the MMU **12** can consist of a collection of MMU specific applications running on distinct servers in order to avoid a single point of failure, address availability requirements, and handle a high volume of requests. In this example, each individual server portion of the MMU **12** operates in conjunction with other server portions of the MMU **12** to redirect service requests to another server portion of the MMU **12.** Additionally, the master adjudicator **74** assigns redirected service requests to another server portion of the MMU **12,** prioritizing each request and also ensuring that each request is processed.

With reference to **FIGS. 2** & **3****,** the managing units **64-72** each include separate features and rules to govern their operation. For example, the asset manager **64** governs the execution of the Monitor Pumps **52** and Monitor Lines **54** programs; the drug library manager **68** governs the execution of the Drug Library **42** and Download Drug Library **44** programs; the therapy manager **72** governs the execution of the Process Drug Order **46,** Maintain Expert Clinical Rules **48,** and Apply Expert Clinical Rules **50** programs; and the clinical data manager **73** governs the execution of the Generate Reports **56** and View Data **58** programs. Other distribution of the functional MMU programs **42-62** among the managing units **64-73** can be made in accordance with the present invention.

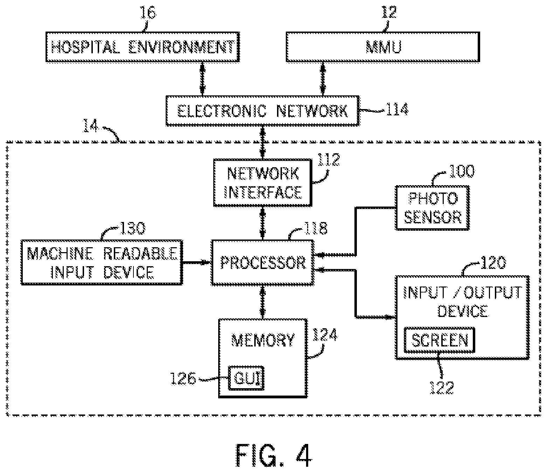

**FIG. 4** is a schematic diagram of a medical device, in accordance with the present invention. An electronic network **114** connects the MMU **12,** medical device **14,** and hospital environment **16** for electronic communication. The electronic network **114** can be a completely wireless network, a completely hard wired network, or some combination thereof. As used herein, the term "medical device" includes without limitation a device that acts upon a cassette, reservoir, vial, syringe, or tubing to convey medication or fluid to or from a patient (for example, an enteral pump, a parenteral infusion pump, a patient controlled analgesia (PCA) or pain management medication pump, or a suction pump), a monitor for monitoring patient vital signs or other parameters, or a diagnostic, testing or sampling device.

The pump style medical device **14** includes a network interface **112** for connecting the medical device **14** to electronic network **114.** Where a wireless connection to the electronic network **114** is desired, network interface **112** operates an antenna for wireless connection to the electronic network **114.** The antenna can project outside the medical device 14 or be enclosed within the housing of the device.

A processor **118** is included in the medical device **14,** includes a real time clock (not shown), and performs various operations described in greater detail below. The input/output device **120** allows the user to receive output from the medical device **14** and/or input information into the medical device **14.**

Those skilled in the art will appreciate that input/output device **120** can be provided as a single device such as a touch screen **122,** or as a separate display device and a separate input device (not shown). In one embodiment, the display screen **122** of the medical device **14** is a thin film transistor active matrix color liquid crystal display with a multi-wire touch screen. A membrane generally impermeable to fluids overlays the display screen **122** so the user can press images of keys or buttons on the underlying screen with wet gloves, dry gloves, or without gloves to trigger an input.

A memory **124** communicates with the processor **118** and stores code and data necessary for the processor **118** to perform the functions of the medical device **14.** More specifically, the memory **124** stores multiple programs formed in accordance with the present invention for various functions of the medical device **14** including a graphical user interface program **126** with multiple subparts described in greater detail below.

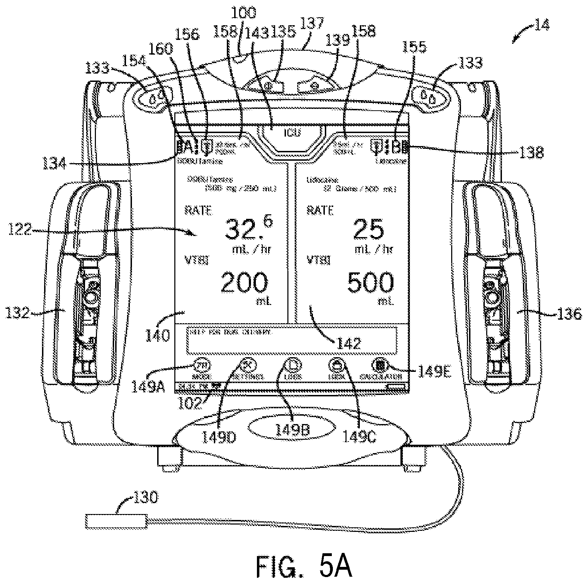

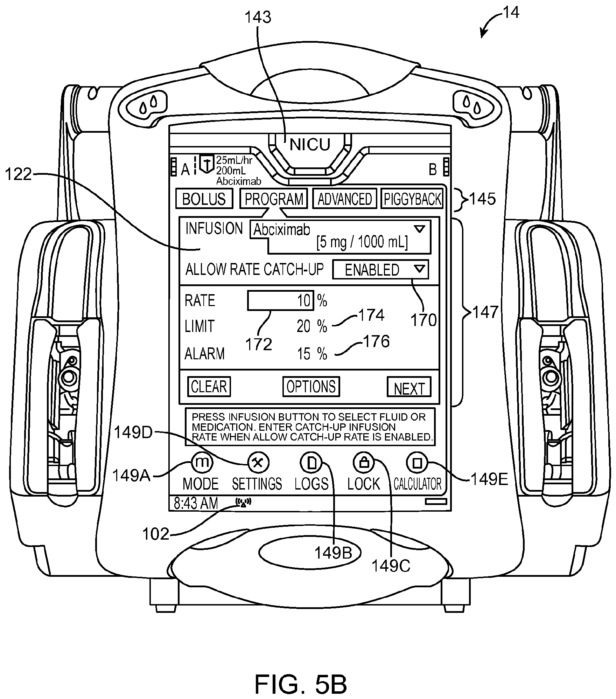

**FIGS. 5A** **&** **5B** are perspective views of a multi-channel medical device in communication with a machine-readable input device, in accordance with the present invention. **FIG. 5A** illustrates an infusion pump with a split screen display, having one portion associated with each channel. **FIG. 5B** illustrates an infusion pump with a screen display for receiving infusion programmable input from a user. The medical device **14** in this example is a multi-channel infusion pump. Those skilled in the art will appreciate that the medical device **14** can be a single channel infusion pump, a multi-channel infusion pump (as shown), a combination thereof, or the like, as desired for a particular application.

Referring to **FIG. 5A****,** the medical device **14** provides a machine-readable input device **130.** The machine-readable input device **130** communicates with the medical device **14** to input machine-readable information to the medical device **14.** The machine-readable input device **130** can communicate, directly or indirectly, with the medical device **14** via a wireless or hard-wired connection. The machine-readable input device **130** can be a device that is separate from but associated or in communication with the medical device **14.** The machine-readable input device **130** can be any sort of data input means, including those adapted to read machine-readable indicia, such as a barcode scanner or handheld personal digital assistant (PDA). Alternatively, the machine-readable input device **130** can be operable to read in other known forms of machine-readable information, such as radio frequency identification tags (RFID), touch memory, digital photography, biometrics, etc.

The medical device **14** is a multi-channel pump having a first channel **132** with first channel machine-readable label **134** and a second channel **136** with a second channel machine-readable label **138.** A user of the medical device **14** operates the machine-readable input device **130** to select a channel from one or more channels **132** and **136,** by scanning in the associated machine-readable label **134** or **138.**

The user selects the desired channel **132** or **136** by using the machine-readable input device **130** to scan a factory or hospital programmed, unique, machine-readable label **134** or **138** that is electronically generated and presented on the screen **122,** preferably positioned near the respective channel **132** or **136.** Alternatively, the machine-readable labels **134** and **138** are physically affixed to the medical device **14,** preferably on or positioned near the channel **132** and **136,** respectively. Since the machine-readable labels **134** and **138** are generated and/or can be stored in memory **124** by the medical device **14,** the medical device **14** can associate the machine-readable labels **134** and **138** to the channels **132** or **136.** The medical device **14** then allows the user to program and activate the selected channel **132** or **136.** The user may also manually select the desired channel by touching an appropriate folder tab on the touch screen. The folder tabs are labeled and/or physically arranged on the screen so as to be proximate to the corresponding channel **132** or **136.**

In a further aspect of the wireless embodiment, all the medical devices can periodically broadcast a unique wireless device/channel IP address and/or a self-generated unique machine-readable label (for example, a barcode) **134** or **138** that can also be presented on the screen **122.** Alternatively, the machine-readable labels **134** and **138** are physically affixed to or posted on the medical device **14.** Each medical device will correlate such broadcasted or posted device/channel IP addresses and/or barcodes with a particular patient, who is also identified by a unique machine readable label (not shown) or patient IP address. The user associates the desired pump(s) or channel(s) **132, 136** with the patient by using the machine-readable input device **130** to scan the unique machine-readable labels **134, 138** and the patient's machine readable label. This causes the appropriate pump processor(s) **118** to associate the appropriate pump channel(s) **132, 136** with the patient. Then the pumps or channels can associate, communicate, and coordinate with each other wirelessly.

The graphical user interface program reallocates screen **122** for the medical device **14.** Specifically, **FIG. 5A** illustrates a multi-channel infusion medical device **14** with a split touch screen **122** having a first channel screen portion **140** associated with first channel **132** and a second channel screen portion **142** associated with the second channel **136.** Each channel screen portion **140** and **142** presents a subset of the delivery information regarding the respective channels **132** or **136** including without limitation therapeutic agent name, concentration, dose rate, VTBI, and alarm information, in a font size that it is easily readable by a user from a distance such as, for example, from approximately fifteen to twenty feet (4.6-6.2 meters) away. This is what is defined herein as a "far view" delivery screen. The far view delivery screens display subsets of the information found on the relevant "near view" delivery screens. The near view delivery screen displays information such as, drug name, concentration, dose rate, time remaining, VTBI, volume remaining, and alarm name for the highest priority alarm if in an alarm state.

In practice, the delivery screen displays a near view when the user is programming the device as illustrated by **FIG. 5B****.** The near view delivery screen will switch to the far view delivery screen after a predetermined period of time that is predetermined by the manufacturer, configurable by the facility via the drug library, and/or set by the caregiver at the pump, for example after 20 seconds. Often, the user does not want to wait for the predetermined length of time to view the far screen.

Returning to **FIG. 5A****,** the channel screen portion **140** or **142** selected or corresponding to the tab selected expands in area but the size of at least some of the text therein is shrunk. The shrinkage of one of the channel screen portions **140** and **142** and enlargement of its counterpart provides additional space for one or more data display or data entry fields to be placed on screen **122,** as shown in **FIG. 5B****.** As discussed below, data displays or data entry fields are placed on screen **122** in space previously occupied by portions of the channel screen portion **140** or **142.** This reallocation of space on screen **122** permits the user to enter inputs more easily since the data entry field can be large, preferably at least as large or, more preferably, larger in area than the original channel screen portions **140** and **142** were in the delivery screen mode. Additionally, the reallocation of space on screen **122** provides greater space for presenting information on the channel being adjusted or monitored.

Referring again to **FIG. 5A****,** the medical device **14** includes dedicated or fixed tactile infuser buttons, and images of buttons on the LCD-touch screen **122.** The fixed tactile buttons **133, 135, 137**, and **139** provide the following functions: LOAD/EJECT button **133**--opens and closes the cassette carriage; ON/OFF button **135**--turns power on and off; ALARM SILENCE button **137**--silences a silenceable alarm for a specified period of time, for example two minutes; and EMERGENCY STOP button **139**--stops all channels. The LCD color touch screen **122** allows the user to access and use on-screen button images, for example **3**D button images, and data entry fields. The touch screen **122** uses a membrane over the LCD display so a single keypress does not cause significant infusion pole movement nor is it mistaken for a double keypress. The touch screen also accommodates a keypress whether the user is wearing wet gloves, dry gloves, or no gloves.

LCD touch screen button images **143, 145, 147,** and **149A-149E** are located as shown in **FIGS. 5A** **&** **5B** perform the following functions: Patient Information Tab **143**--displays the clinical care area, preselected patient information (including without limitation name, ID number, etc.), and provides access to a more detailed patient information screen; Channel Level Therapy Buttons **145**--accessed by button images on the infuser touch screen, are used to select an infusion therapy; Program Level Buttons **147**--accessed by pressing areas, drop-down list triangles, boxes or text boxes on the programming screen, are used to select dose parameters of an infusion; and Device Level Buttons **149A-149E** at the bottom of the touch screen are used to display and control device level features, including without limitation Mode **149A** (for example, Operational or Biomed), Logs **149B,** Locks **149C,** Settings **149D,** and Calculator display **149E.** A wireless indicator image **102** displayed at the bottom of the screen **122** indicates that the medical device 14 is connected and ready for communication.

By using the Channel Level Therapy Buttons **145** and the Program Level Buttons **147,** the healthcare practitioner can program each individual channel of the pump with specific fluid therapies in a variety of weight- and body surface area-based units such as micrograms/kg/hour, grams/m²/hr, and other delivery specifications for the following modes: Basic Therapy--includes dose calculation, which allows dose rate programming based on volume to be infused (VTBI), drug amount, infusion time and drug concentration and simple rate programming that allows programming of volumetric rate (mL/hr) based upon VTBI and time; Bolus delivery ---allows user to program a single uninterrupted discrete delivery based on dose amount and time (the bolus can be delivered from the primary or a secondary container); Piggyback delivery--allows user to program the delivery of a secondary infusion, to be delivered through the same cassette as the primary infusion (the primary infusion is paused until the piggyback VTBI completes); and Advanced Programming. Advanced Programming mode provides various types of programs including: Multistep--which allows a sequential delivery of fluid in up to 10 steps, with fluid volumes and delivery rates programmable for each step based on Rate and Volume or Volume and Time; Variable Time--which allows up to 24 dose calculation steps at specified clock times; Intermittent--a calculated dose or step to be delivered at regular intervals; and Taper--a delivery that ramps up and/or ramps down to a plateau rate.

Referring to **FIGS. 4** & **5A****,** the graphical user interface **126** provides channel indicators presented on screen **122.** The channel indicators associate on-screen programming, delivery, and alarm information with a particular delivery channel by using graphical depictions such as a channel indication icon **154, 155.** The channel indication icon **154** or **155** is a graphical item clearly associating on-screen programming, delivery, and alarm information with a specified associated delivery channel. The channel indication icons **154** and **155** are located on a tab **158** associated with a specified delivery channel of the medical device The channel indication icon **154** or **155** may include but is not limited to a user readable letter or number, a machine-readable indicator **134,** or a combination thereof. The graphical user interface program **126** also provides a drip indicator icon **160** and an infusion status icon **156** presented on screen **122.**

Referring to **FIG. 5B****,** the screen **122** provides an optional drop-down box **170** for setting an Allow Rate Catch-Up flag to one of an Enabled setting and a Disabled setting at the pump. The drop-down box **170** allows the user to enable or disable the rate catch-up function and to override the default rate catch-up flag provided in the drug library, if desired. When the Allow Rate Catch-Up flag is set to the Enabled setting, the user can enter a catch-up rate factor in the catch-up rate factor value box **172.** In this example, the screen **122** also displays a catch-up rate factor limit value **174** and a catch-up rate factor alarm value **176** provided through the drug library. The catch-up rate factor limit value **174** is the maximum catch-up rate factor which the user can enter in the catch-up rate factor value box **172,** i.e., the maximum catch-up rate factor or hard limit allowed for the particular therapeutic agent. The catch-up rate factor alarm value **176** is a soft limit on the catch-up rate factor. In one example, the screen **122** will provide an alarm when the user enters a value in the catch-up rate factor value box **172** which exceeds the catch-up rate factor alarm value **176,** but the infusion pump will accept the catch-up rate factor after the user acknowledges the alarm or indicates a decision to override the soft limit as long as the catch-up rate factor does not exceed the hard limit or catch-up rate factor limit value **174.** Those skilled in the art will appreciate that the catch-up rate factor limit value **174** and the catch-up rate factor alarm value **176** can be omitted or set to a high value as desired for a particular application. In one embodiment, the default values for the Allow Rate Catch-Up flag setting, catch-up rate factor value box **172,** catch-up rate factor limit value **174,** and/or catch-up rate factor alarm value **176** can be loaded into the infusion pump from a remote computer as part of a drug library editing program such as HOSPIRA MEDNET™ software. In another embodiment, the default values for the Allow Rate Catch-Up flag, catch-up rate factor value box **172,** catch-up rate factor limit value **174,** and/or catch-up rate factor alarm value **176** can be loaded into the infusion pump by the user at the infusion pump. In another hybrid embodiment, the default values can be established in a drug library downloaded to the pump and, if allowed by a setting in the drug library, later overridden or modified by the user at the pump. In another embodiment, the entire catch-up rate behavior can be predetermined by the manufacturer and hard coded into the pump, without any user customization being allowed.

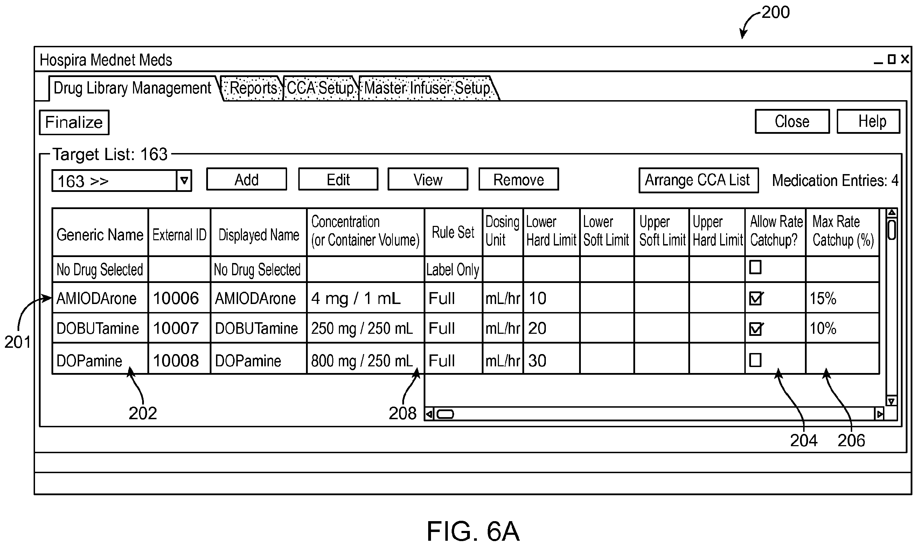

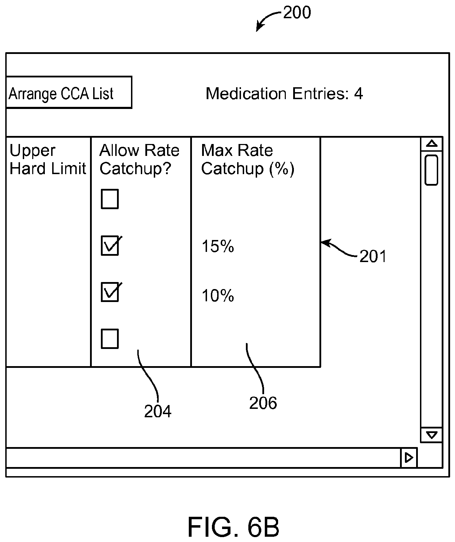

**FIGS. 6A** **&** **6B** are screen shots of a graphical user interface and detail of the graphical user interface, respectively, for configuring a drug library, in accordance with the present invention. The graphical user interface **200** can be displayed on the display/input device **38** of the MMU **12** (as shown in **FIG. 2**) and used to receive data for creating or updating the drug library, such as the catch-up rate factor, Allow Rate Catch-up flag setting, maximum catch-up rate factor setting, maximum catch-up rate factor alarm setting, and the like.

Referring to **FIGS. 6A** **&** **6B****,** the graphical user interface **200** includes a table **201** to receive different drugs and therapeutic agents in the drug library database. Drug list **202** includes a list of the names of the drugs in the drug library, which could be the generic names, brand names or both. The drug list can include multiple entries for the same drug but different concentrations or clinical uses (cardiac, renal, pediatric). The Allow Rate Catch-Up Flag list **204** includes the allow rate catch-up flag setting for each drug/concentration/use entry ("drug entry" for short) in the drug list **202** to determine whether rate catch-up is allowed for the particular drug entry. In addition, the Allow Rate Catch-up flag setting can be a function of pump type and/or clinical care area location. The maximum rate catch-up list **206** includes the maximum catch-up rate factor setting for each drug entry in the drug list **202** for which rate catch-up is allowed. The maximum catch-up rate factor setting also can be a function of pump type and clinical care area location. In one embodiment, allowable maximum catch-up rate factors are regular linear percentages at predetermined intervals, e.g., 5%, 10%, 15%, 20%, et *cetera.* The table **201** can also include other parameters that constrain or limit the drug maximum catch-up rate such as the normal global constraints on rate already configured via the MMU 12 and HOSPIRA MEDNET™ software (Lower Hard Limit, Lower Soft Limit, Upper Soft Limit, and/or Upper Hard Limit). The maximum catch-up rate factor alarm setting and other drug maximum catch-up rate limits for each drug entry also can be a function of pump type and clinical care area location.

The catch-up rate factor is a simple percentage applied to the desired infusion rate to obtain a catch-up infusion rate when the actual accumulated infusion volume is less than the expected accumulated infusion volume. In some cases the desired infusion rate is input directly as a rate or volume per unit of time, such as mL/hr. In other cases the desired infusion rate is a calculated value based upon a dose and the weight or body surface area of the patient. For example, a dose of 10 mL/kg/hr can be prescribed for a patient who weighs 100 kg. Thus, the desired infusion rate would be calculated as 1000 mL/hr. In other cases the desired infusion rate is calculated based upon the dose and concentration of drug in the container. For example, if a dose of 10 mcg/hr is prescribed to be delivered from a 1000 mL container of fluid that has a concentration of 100 mcg of the drug, then the desired infusion rate is calculated at 100 mL/hr. There are other alternative dosing units that are known in the art to provide calculated desired infusion rates. In one embodiment, the catch-up rate factor is added to the desired infusion rate. In another embodiment, the catch-up rate factor (for example 1.05) is multiplied by the desired infusion rate. In one embodiment, allowable catch-up rate factors are regular linear percentages at predetermined intervals, e.g., 5%, 10%, 15%, 20%, *et cetera* to make it easier for the user to select a catch-up rate factor. The catch-up rate factor applies a linear adjustment to the desired infusion rate and does not rely on any input from physiological factors of the patient. Thus, the configurable closed loop delivery rate catch-up is straightforward and does not rely on complex algorithms or control schemes. Instead the feedback mechanism of this algorithm is based only on the measured versus expected accumulated volume delivered over time by the pump. The new rate Y is determined by a simple single order equation X + AX or AX; where A equals the catch-up rate factor as described above.

The Allow Rate Catch-up flag setting as a function of pump type can account for different pump types, makes and models, as well as uses for which various pump types are employed. In one example, one pump type using a cartridge and driving a plunger with a stepper motor can be used for general infusion such as saline solutions or the like, so that there is little risk in allowing rate catch-up. In another example, another pump type using a prefilled syringe can be used for analgesics or opiates, so that it may not be desirable to allow rate catch-up. Other pump types may have multiple uses or therapies and it may be desirable to control the enablement of the catch-up rate feature for each of the plurality of uses available with such a pump type.

The Allow Rate Catch-up flag setting can be a function of clinical care area location. In one example, an infusion pump used in a treatment area in which the patients are in serious or critical condition, such as an emergency or operating room, may not want to allow rate catch-up. In another example, an infusion pump used in a treatment area in which the patients are in good condition may want to allow rate catch-up.

Those skilled in the art will appreciate that the table **201** can also include other data as desired for a particular application. The table **201** can include other exemplary columns **208** for additional data for the different drugs, such as External Drug ID Numbers, Drug Display Names, Drug Concentration/Container Volume, Selected Drug Rule Set (Label Only, Limited, Full), Drug Dosing Unit, Drug Dosing Limits (Lower Hard Limit, Lower Soft/Alarm Limit, Upper Soft/Alarm Limit, and/or Upper Hard Limit) or the like.

The drug library provides flexibility for various combinations of parameters as desired for a particular application. The drug library can have different Allow Rate Catch-up flag settings for different drugs or drug entries in the drug library. The drug library can have different maximum permissible catch-up rate factor settings for different drugs in the drug library. The drug library can have a given drug listed in multiple different clinical care areas (CCAs) in the drug library with at least one of different Allow Rate Catch-up flag settings and different maximum catch-up rate factor settings for a the given drug.

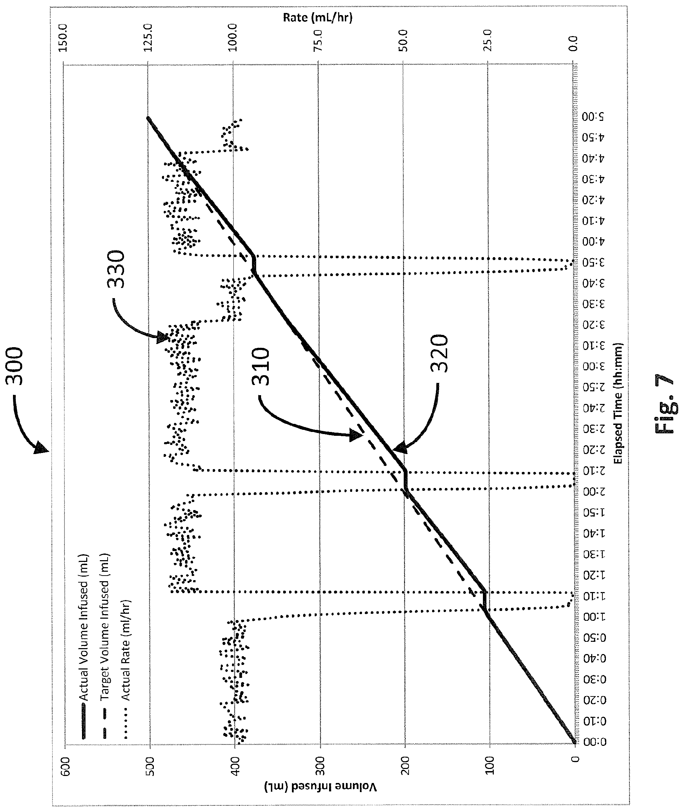

**FIG. 7** is a graph of an infusion volume and infusion rate versus time modeled for an infusion with an infusion pump employing configurable closed loop delivery rate catch-up, in accordance with the present invention. The graph **300** includes the expected accumulated infusion volume **310,** the actual accumulated infusion volume **320,** and the infusion rate **330.**

The expected accumulated infusion volume **310** increases linearly at a desired infusion rate of 100 mL per hour. The actual accumulated infusion volume **320** increases linearly from time 0:00 until time 1:00 at the originally programmed or desired infusion rate **330** of 100 mL per hour. At time 1:00, the infusion is interrupted so that the infusion rate **330** remains approximately zero and the actual accumulated infusion volume **320** remains about 100 mL until time 1:15, when the infusion resumes. At time 1:15, the actual accumulated infusion volume **320** is less than the expected accumulated infusion volume **310,** so the infusion rate is increased by the catch-up rate factor of 15% and the infusion resumes at a catch-up infusion rate of 115 mL per hour from time 1:15 to time 2:00. At time 2:00, the actual accumulated infusion volume **320** has not quite yet caught up with the expected accumulated infusion volume **310** and the infusion is once again interrupted. The infusion rate **330** remains approximately zero and the actual accumulated infusion volume **320** remains about 200 mL until time 2:10, when the infusion resumes. From time 2:10 until time 3:20, the infusion is delivered at the catch-up infusion rate of 115 mL per hour until the actual accumulated infusion volume **320** equals the expected accumulated infusion volume **310** at time 3:20, when the infusion rate **330** is reduced to the originally programmed or desired infusion rate of 100 mL per hour. At time 3:45, the infusion is once again interrupted so that the infusion rate **330** remains approximately 0 and the actual accumulated infusion volume **320** remains at about 375 mL until the infusion resumes at time 3:55. From time 3:55 until time 4:40, the infusion is delivered at the catch-up infusion rate of 115 mL per hour until the actual accumulated infusion volume **320** equals the expected accumulated infusion volume **310** at time 4:40, when the infusion rate **330** is reduced to the originally programmed or desired infusion rate of 100 mL per hour. Thus, the desired accumulated volume of 500 mL has been delivered by the scheduled time 5:00 in spite of three interruptions in the infusion.

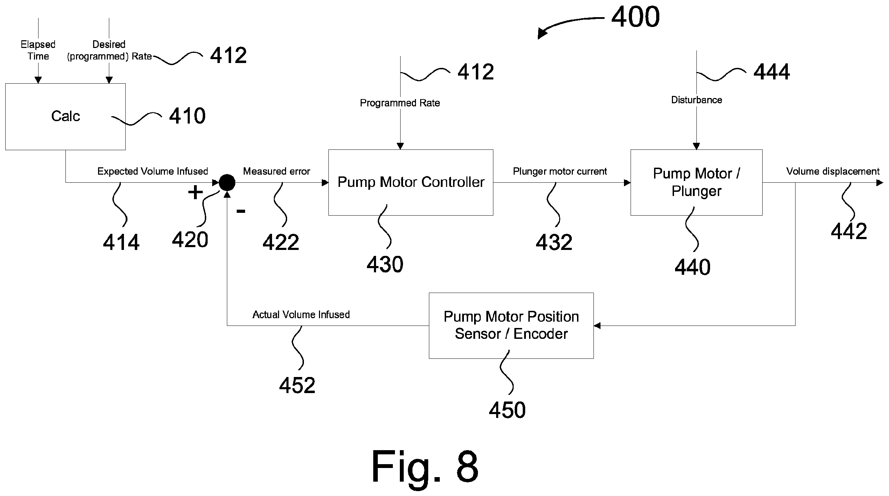

**FIG. 8** is a block diagram of a control model for an infusion pump employing configurable closed loop delivery rate catch-up, in accordance with the present invention. The control model **400** includes an infusion volume calculator **410,** a volume comparator **420,** a pump controller **430,** a pump drive **440,** and a flow integrator **450.** The infusion volume calculator **410** receives a desired infusion rate signal **412** and generates an expected accumulated infusion volume signal **414** from the originally programmed desired infusion rate signal **412** and the elapsed time. The volume comparator **420** receives the expected infusion volume signal **414** and an actual accumulated infusion volume signal **452,** and generates a volume error signal **422** from the expected accumulated infusion volume signal **414** and the actual accumulated infusion volume signal **452.** The pump controller **430** also receives the desired infusion rate signal **412** and the accumulated volume error signal **422,** and generates a pump drive signal **432** from the desired infusion rate signal **412** and the accumulated volume error signal **422.** The pump drive **440** receives the pump drive signal **432** to deliver the infusion **442.** For modeling purposes, the pump drive **440** is subject to disturbances **444** which can cause or result in interrupted delivery of the infusion **442.** The disturbance may include but are not limited to stoppages due to alarms, occlusions and other faults. The flow integrator **450** is operable to monitor the pump drive **440** and/or the infusion **442** and generate the actual accumulated infusion volume signal **452.** In one embodiment, the pump drive **440** moves the plunger in a syringe and the flow integrator **450** senses pump drive/plunger position. In another embodiment, the pump drive **440** is a stepper motor and the flow integrator **450** counts pump strokes or motor steps. In yet another embodiment, the pump drive **440** is a rotary pump and the flow integrator 450 counts pump rotations. The present invention could also be applied with a drip counting device to provide the necessary feedback concerning the actual flow rate and/or accumulated volume. Returning to discussion of the embodiment for driven pumps, the pump drive signal **432** is a function of the desired infusion rate signal **412** multiplied by a catch-up rate factor when the volume error signal **422** meets (equals and/or exceeds) a threshold that indicates that actual accumulated infusion volume is less than expected accumulated infusion volume, to catch up interrupted delivery of an infusion. The pump drive signal **432** is a function of the desired infusion rate signal **412** alone or returns to the originally programmed or set rate when the accumulated volume error signal **422** indicates that the actual accumulated infusion volume is greater than or equal to the expected accumulated infusion volume.

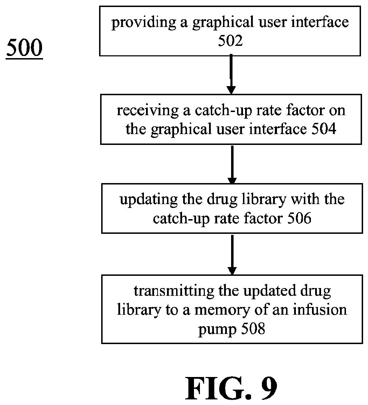

**FIG. 9** is a flowchart of a method for configuring a drug library for use with an infusion system employing configurable closed loop delivery rate catch-up, in accordance with the present invention. The method **500** includes providing a graphical user interface **502** for modifying a drug library of the medication management unit; receiving a catch-up rate factor on the graphical user interface **504;** updating the drug library with the catch-up rate factor **506;** and transmitting the updated drug library to a memory of an infusion pump **508.** The method **500** can be performed on a medication management unit having a processing unit and a storage medium coupled to the processing unit, the storage medium containing programming code executable by the processing unit to perform the steps of the method **500.**

Those skilled in the art will appreciate that the drug library can include additional settings for a method for configuring a drug library for use with an infusion system employing configurable closed loop delivery rate catch-up as desired for a particular application. In one embodiment, the drug library can further include an Allow Rate Catch-up flag setting having one of an Enabled setting and a Disabled setting, the Allow Rate Catch-up flag setting being a function of a parameter selected from the group consisting of drug/concentration/use entry, pump type, and clinical care area location. In another embodiment, the drug library can further include a maximum catch-up rate factor setting having a numerical value, the maximum catch-up rate factor setting being a function of a parameter selected from the group consisting of drug entry, pump type and clinical care area location. In yet another embodiment, the drug library can further include a maximum catch-up rate factor alarm setting having a numerical value, the maximum catch-up rate factor alarm setting being a function of a parameter selected from the group consisting of drug entry, pump type and clinical care area location.

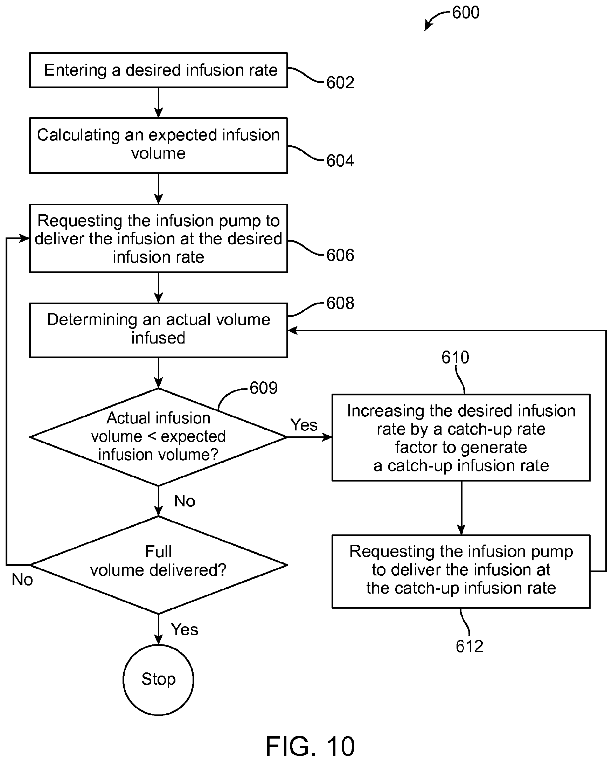

**FIG. 10** is a flowchart of a method for operating an infusion pump employing configurable closed loop delivery rate catch-up, in accordance with the present invention. The method **600** includes entering a desired infusion rate **602** for the infusion pump; calculating an expected accumulated infusion volume **604** as a function of time from the infusion rate; requesting the infusion pump to deliver the infusion at the desired infusion rate **606;** determining an actual accumulated infusion volume **608** at a particular time; and determining whether the actual accumulated infusion volume is less than the expected accumulated infusion volume **609.** When the actual accumulated infusion volume is less than the expected accumulated infusion volume, the method **600** continues with increasing the desired infusion rate by a catch-up rate factor to generate a catch-up infusion rate **610** and requesting the infusion pump to deliver the infusion at the catch-up infusion rate **612.** When the actual accumulated infusion volume is not less than the expected accumulated infusion volume, the method **600** continues monitoring the output of the pump and with delivering the infusion at the originally programmed or desired infusion rate. The method **600** can also request the infusion pump to deliver the infusion at the originally programmed or desired infusion rate after delivery of the infusion at the catch-up infusion rate **612** when the actual accumulated infusion volume is equal to the expected accumulated infusion volume. Once the actual accumulated infusion volume is equal to or greater than the expected accumulated infusion volume, the method 600 continues by determining whether the full accumulated volume prescribed or programmed has been delivered or not. The method 600 stops if the full volume has been delivered, or loops back to continue with step 606 if more volume remains to be delivered.

The method **600** can also allow the user at the infusion pump to provide input. The method **600** can include a user at the infusion pump inputting an Allow Rate Catch-up flag setting to a Disabled setting to disable the increasing and the delivery rate catch-up function. The method **600** can include the user at the infusion pump inputting the catch-up rate factor to a desired value. In one embodiment, the catch-up rate factors are regular linear percentages at predetermined intervals, e.g., 5%, 10%, 15%, 20%, *et cetera.* In other embodiments, the drug library editor or the pump may allow the user more flexibility to customize the values and the intervals between them.

The method **600** may or may not require user action before applying the catch-up infusion rate if desired for a particular application. In one embodiment, the requesting the infusion pump to deliver the infusion at the catch-up infusion rate **612** can occur automatically, without user action, by increasing the desired infusion rate by a catch-up rate factor to generate a catch-up infusion rate **610.** In another embodiment, the method **600** can include annunciating an alarm or warning before increasing the desired infusion rate by a catch-up rate factor to generate a catch-up infusion rate **610,** and requiring the user acknowledge the alarm and confirm that the catch-up rate behavior is desired before the requesting the infusion pump to deliver the infusion at the catch-up infusion rate **612.** In another embodiment, the method **600** can include annunciating an alarm or warning after increasing the desired infusion rate by a catch-up rate factor to generate a catch-up infusion rate **610,** and allowing the user to confirm or reject the catch-up rate behavior. If the catch-up rate behavior is rejected, the infusion will revert to the originally programmed infusion rate.

The method **600** can also provide limits and alarms in response to user input. In one embodiment, the method **600** further includes rejecting the input catch-up rate factor when the desired value is greater than a maximum catch-up rate factor setting. In another embodiment, the method **600** further includes providing an alarm when the desired value is greater than a maximum catch-up rate factor alarm setting. In yet another embodiment, the increasing the desired infusion rate by a catch-up rate factor in the method **600** includes receiving an alarm when at the particular time the actual accumulated infusion volume is less than the expected accumulated infusion volume; and acknowledging the alarm prior to increasing the desired infusion rate by a catch-up rate factor to generate a catch-up infusion rate.

The method **600** can be performed on an infusion pump having a processor and the memory coupled to the processor, the memory containing programming code executable by the processor to perform the steps of the method **600.** In one embodiment, the infusion pump 14 can be in electronic communication with a medication management unit 12 and the catch-up rate factor can be part of an updated drug library transmitted from the medication management unit 12 and received at the medical device 14.

Terms of equality and inequality (less than, greater than) as used herein as commonly used in the art, i.e., accounting for uncertainties present in measurement and control systems. Thus, such terms can be read as approximately equal, approximate less than, and/or approximately greater than. In one example, two values can be considered equal when they are within 5% of each other. In other aspects of the invention, an acceptable threshold of deviation or hysteresis can be established by the pump manufacturer, the editor of the drug library, or the user at the pump.

While the embodiments of the invention disclosed herein are presently considered to be preferred, various changes and modifications can be made without departing from the scope of the invention. The scope of the invention is defined by the appended claims

## Claims

1. An infusion pump (14) with catch-up of interrupted delivery of an infusion, the infusion pump comprising:
a processor (118);
a memory (124) coupled to the processor (118), the memory (124) containing programming code to:
receive an updated drug library with a catch-up rate factor,
receive a desired infusion rate (330) from the user at the infusion pump (14);
calculate an expected accumulated infusion volume (310) as a function of time from the desired infusion rate (330);
request delivery of the infusion at the desired infusion rate (330);
determine an actual accumulated infusion volume (320) at a particular time;
increase the desired infusion rate (330) by the catch-up rate factor to generate a catch-up infusion rate when at the particular time the actual accumulated infusion volume (320) is less than the expected accumulated infusion volume (310); and
request delivery of the infusion at the catch-up infusion rate.

2. The infusion pump of claim 1 wherein the memory (124) further contains programming code to request the infusion pump (14) to deliver the infusion at the desired infusion rate (330) after delivery of the infusion at the catch-up infusion rate when the actual accumulated infusion volume (320) is equal to the expected accumulated infusion volume (310).

3. The infusion pump of claim 1 wherein the memory (124) further contains programming code to receive input of an Allow Rate Catch-up flag setting from a user at the infusion pump.

4. The infusion pump of claim 1 wherein the memory (124) further contains programming code to receive input of a maximum permissible catch-up rate factor from a user at the infusion pump or from a remote computer.

5. An infusion system comprising an infusion pump (14) according to claim 1 and:
a medication management unit (12) having a processing unit (36) and a storage medium (40) coupled to the processing unit (36), the storage medium (40) containing programming code executable by the processing unit (36) to:
provide a graphical user interface (200) for modifying a drug library of the medication management unit (12);
receive a catch-up rate factor on the graphical user interface (200);
update the drug library with the catch-up rate factor; and
transmit the updated drug library to a memory (124) of the infusion pump; and
the infusion pump (14) being in electronic communication with the medication management unit (12).

6. The infusion system of claim 5 wherein the drug library further includes an Allow Rate Catch-up flag setting having one of an Enabled setting and a Disabled setting, the Allow Rate Catch-up flag setting being a function of a parameter selected from the group consisting of drug entry, pump type, and clinical care area location.

7. The infusion system of claim 5 wherein the drug library further includes a maximum catch-up rate factor setting having a numerical value, the maximum catch-up rate factor setting being a function of a parameter selected from the group consisting of drug entry, pump type, and clinical care area location.

8. The infusion system of claim 5 wherein the drug library further includes a maximum catch-up rate factor alarm setting having a numerical value, the maximum catch-up rate factor alarm setting being a function of a parameter selected from the group consisting of drug entry, pump type, and clinical care area location.

## Patentansprüche

1. Infusionspumpe (14) mit Aufholung einer unterbrochenen Abgabe einer Infusion, wobei die Infusionspumpe umfasst:
einen Prozessor (118);
einen mit dem Prozessor (118) gekoppelten Speicher (124), wobei der Speicher (124) Programmiercode enthält, um:
eine aktualisierte Arzneimittelbibliothek mit einem Aufholratenfaktor zu erhalten,
eine gewünschte Infusionsrate (330) von dem Benutzer an der Infusionspumpe (14) zu erhalten;
ein erwartetes akkumuliertes Infusionsvolumen (310) als Funktion der Zeit aus der gewünschten Infusionsrate (330) zu berechnen;
die Abgabe der Infusion mit der gewünschten Infusionsrate (330) anzufordern;
ein tatsächliches akkumuliertes Infusionsvolumen (320) zu einem bestimmten Zeitpunkt zu bestimmen;
die gewünschte Infusionsrate (330) um den Aufholratenfaktor zu erhöhen, um eine Aufhol-Infusionsrate zu erzeugen, wenn zu dem bestimmten Zeitpunkt das tatsächliche akkumulierte Infusionsvolumen (320) geringer ist als das erwartete akkumulierte Infusionsvolumen (310); und
die Abgabe der Infusion mit der Aufhol-Infusionsrate anzufordern.

2. Infusionspumpe nach Anspruch 1, wobei der Speicher (124) weiter Programmiercode enthält, um die Infusionspumpe (14) aufzufordern, die Infusion mit der gewünschten Infusionsrate (330) nach Abgabe der Infusion mit der Aufhol-Infusionsrate abzugeben, wenn das tatsächliche akkumulierte Infusionsvolumen (320) gleich dem erwarteten akkumulierten Infusionsvolumen (310) ist.

3. Infusionspumpe nach Anspruch 1, wobei der Speicher (124) weiter Programmiercode enthält, um eine Eingabe einer Merkereinstellung "Aufholrate zulassen" von einem Benutzer an der Infusionspumpe zu empfangen.

4. Infusionspumpe nach Anspruch 1, wobei der Speicher (124) weiter Programmiercode enthält, um die Eingabe eines maximal zulässigen Aufholratenfaktors von einem Benutzer an der Infusionspumpe oder von einem entfernten Computer zu empfangen.

5. Infusionssystem umfassend eine Infusionspumpe (14) nach Anspruch 1 und:
eine Medikamentenverwaltungseinheit (12) aufweisend eine Verarbeitungseinheit (36) und ein mit der Verarbeitungseinheit (36) gekoppeltes Speichermedium (40), wobei das Speichermedium (40) Programmiercode enthält, der von der Verarbeitungseinheit (36) ausführbar ist, um:
eine graphische Benutzerschnittstelle (200) zum Modifizieren einer Arzneimittelbibliothek der Medikamentenverwaltungseinheit (12) bereitzustellen;
einen Aufholratenfaktor auf der graphischen Benutzeroberfläche (200) zu erhalten;
die Arzneimittelbibliothek mit dem Aufholratenfaktor zu aktualisieren; und
die aktualisierte Arzneimittelbibliothek an einen Speicher (124) der Infusionspumpe zu übertragen;
und
wobei die Infusionspumpe (14) in elektronischer Kommunikation mit der Medikationsverwaltungseinheit (12) ist.

6. Infusionssystem nach Anspruch 5, wobei die Arzneimittelbibliothek weiter eine Merkereinstellung "Aufholrate zulassen" einschließt, die entweder eine Einstellung "Aktiviert" oder eine Einstellung "Deaktiviert" aufweist, wobei die Merkereinstellung "Aufholrate zulassen" eine Funktion eines Parameters ist, der aus der Gruppe ausgewählt ist, die aus Arzneimitteleintritt, Pumpentyp und Standort des klinischen Versorgungsbereichs besteht.

7. Infusionssystem nach Anspruch 5, wobei die Arzneimittelbibliothek weiter eine Einstellung für den maximalen Aufholratenfaktor einschließt, der einen numerischen Wert aufweist, wobei die Einstellung des maximalen Aufholratenfaktors eine Funktion eines Parameters ist, der aus der Gruppe ausgewählt ist, die aus Arzneimitteleintritt, Pumpentyp und Standort des klinischen Versorgungsbereichs besteht.

8. Infusionssystem nach Anspruch 5, wobei die Arzneimittelbibliothek weiter eine Alarmeinstellung für den maximalen Aufholratenfaktor einschließt, der einen numerischen Wert aufweist, wobei die Alarmeinstellung für den maximalen Aufholratenfaktor eine Funktion eines Parameters ist, der aus der Gruppe ausgewählt ist, die aus Arzneimitteleintritt, Pumpentyp und Lage des klinischen Versorgungsbereichs besteht.

## Revendications

1. Pompe à perfusion (14) avec rattrapage d'administration interrompue d'une perfusion, la pompe à perfusion comprenant :
un processeur (118) ;
une mémoire (124) couplée au processeur (118), la mémoire (124) contenant un code de programmation pour :
recevoir une pharmacothèque mise à jour avec un facteur de vitesse de rattrapage,
recevoir une vitesse de perfusion souhaité (330) de l'utilisateur au niveau de la pompe de perfusion (14) ;
calculer un volume de perfusion accumulé attendu (310) en fonction du temps à partir de la vitesse de perfusion souhaité (330) ;
demander l'administration de la perfusion à la vitesse de perfusion souhaitée (330) ;
déterminer un volume de perfusion accumulé réel (320) à un moment donné ;
augmenter la vitesse de perfusion souhaitée (330) par le facteur de vitesse de rattrapage pour générer une vitesse de perfusion de rattrapage lorsqu'au moment donné, le volume de perfusion accumulé réel (320) est inférieur au volume de perfusion accumulé attendu (310) ; et
demander l'administration de la perfusion à la vitesse de perfusion de rattrapage.

2. Pompe à perfusion selon la revendication 1 dans laquelle la mémoire (124) contient en outre un code de programmation pour demander à la pompe à perfusion (14) d'administrer la perfusion à la vitesse de perfusion souhaitée (330) après l'administration de la perfusion à la vitesse de perfusion de rattrapage lorsque le volume de perfusion accumulé réel (320) est égal au volume de perfusion accumulé attendu (310).

3. Pompe à perfusion selon la revendication 1 dans laquelle la mémoire (124) contient en outre un code de programmation pour recevoir l'entrée d'un paramétrage du drapeau d'autorisation de rattrapage de la vitesse d'un utilisateur au niveau de la pompe à perfusion.

4. Pompe à perfusion selon la revendication 1 dans laquelle la mémoire (124) contient en outre un code de programmation pour recevoir l'entrée d'un facteur de vitesse de rattrapage maximale autorisée d'un utilisateur au niveau de la pompe à perfusion ou d'un ordinateur à distance.

5. Système de perfusion comprenant une pompe à perfusion (14) selon la revendication 1 et :
une unité de gestion des médicaments (12) munie d'une unité de traitement (36) et d'un support d'enregistrement (40) couplés à l'unité de traitement (36), le support d'enregistrement (40) contenant le code de programmation exécutable par l'unité de traitement (36) pour :
fournir une interface utilisateur graphique (200) permettant de modifier une pharmacothèque de l'unité de gestion des médicaments (12) ;
recevoir un facteur de vitesse de rattrapage sur l'interface utilisateur graphique (200) ;
mettre à jour la pharmacothèque avec le facteur de vitesse de rattrapage ; et
transmettre la pharmacothèque mise à jour à la mémoire (124) de la pompe à perfusion ; et
la pompe à perfusion (14) étant en communication électronique avec l'unité de gestion des médicaments (12).

6. Système de perfusion selon la revendication 5 dans lequel la pharmacothèque inclut en outre un paramétrage du drapeau d'autorisation de rattrapage de la vitesse doté d'un élément parmi un paramétrage activé et un paramétrage désactivé, le paramétrage du drapeau d'autorisation de rattrapage de la vitesse étant une fonction d'un paramètre choisi parmi le groupe consistant en entrée de médicaments, type de pompe et emplacement de la zone de soins cliniques.

7. Système de perfusion selon la revendication 5 dans lequel la pharmacothèque inclut en outre un paramétrage du facteur de vitesse de rattrapage maximale présentant une valeur numérique, le paramétrage du facteur de vitesse de rattrapage maximale étant une fonction d'un paramètre choisi parmi le groupe consistant en entrée de médicaments, type de pompe et emplacement de la zone de soins cliniques.

8. Système de perfusion selon la revendication 5 dans lequel la pharmacothèque inclut en outre un paramétrage d'alarme du facteur de vitesse de rattrapage maximale présentant une valeur numérique, le paramétrage d'alarme du facteur de vitesse de rattrapage maximale étant une fonction d'un paramètre choisi parmi le groupe consistant en entrée de médicaments, type de pompe et emplacement de la zone de soins cliniques.
